## Europäisches Patentamt

## European Patent Office

## Office européen des brevets

(11) Veröffentlichungsnummer: **0 275 123**
**A2**

# (12) EUROPÄISCHE PATENTANMELDUNG

(21) Anmeldenummer: 88100545.8

(22) Anmeldetag: 16.01.88

(51) Int. Cl.⁴: **G 01 N 23/223**

(30) Priorität: **16.01.87 DE 3701163**

(43) Veröffentlichungstag der Anmeldung:
**20.07.88 Patentblatt 88/29**

(84) Benannte Vertragsstaaten:
**AT BE CH DE ES FR GB GR IT LI LU NL SE**

(71) Anmelder: **Schnug, Ewald, Dr.**
**Hansastrasse 18**
**D-2300 Kiel 1 (DE)**

(72) Erfinder: **Schnug, Ewald, Dr.**
**Hansastrasse 18**
**D-2300 Kiel 1 (DE)**

(74) Vertreter: **Tönnies, Jan G., Dipl.-Ing.**
**Niemannsweg 133**
**D-2300 Kiel 1 (DE)**

(54) Verfahren zur Bestimmung des Gesamtglucosinolatgehaltes von Rapssamen oder -schrot.

㊗ Verfahren zur Bestimmung des Gesamtglucosinolatgehaltes von Rapssamen oder Rapsschrot, unter Vermahlen des zu untersuchenden Materials mittels eines Messerhomogenisators bis zu einer definierten Korngröße, Tablettieren des vermahlenen Materials bei definiertem Druck, Einbringen der tablettierten Probe in ein hochauflösendes Röntgenspektrometer, Bestrahlen der Probe mit Röntgenstrahlen, Messen der Schwefel-$K_\alpha$-Fluorescenzstrahlung, Berechnen des Schwefelgehalts der Probe aus der Intensität der Schwefel-$K_\alpha$-Strahlen, und Ermitteln des Gesamtglucosinolatgehaltes aus dem berechneten Schwefelgehalt.

EP 0 275 123 A2

# Beschreibung

## Verfahren zur Bestimmung des Gesamtglucosinolatgehaltes von Rapssamen oder -schrot

Die Erfindung betrifft ein Verfahren zur Bestimmung des Gesamtglucosinolatgehaltes von Rapssamen oder Rapsschrot.

Die Qualität von Rapssorten ist wesentlich durch deren Gehalt an Glucosinolaten bestimmt. Rapssamen, der einen festgelegten Glucosinolatgehalt unterschreitet, wird deutlich besser bezahlt als Rapssamen, dessen Glucosinolatgehalt einen vorgegebenen Grenzwert übersteigt. Es ist daher erforderlich, den Gesamtglucosinolatgehalt von Rapssamen exakt zu bestimmen. Die bisher bekannten Verfahren zur Bestimmung des Glucosinolatgehaltes von Rapssaat, nämlich die Gaschromatographie, die Hochdruckflüssigkeitschromatographie, der Palladiumtest und die Reflektometrie genügen den Anforderungen der Praxis nach einem schnellen, unkomplizierten und präzisen Verfahren nicht.

In W. Hartmann (Hrsg.), Meßverfahren unter Anwendung ionisierender Strahlung, Leipzig 1969, S. 307 ff. werden Methodik und Anwendungsbereiche der Röntgenfluoreszenzanalyse dargestellt.

Der Erfindung liegt die Aufgabe zugrunde, ein Verfahren zur Bestimmung des Gesamtglucosinolatgehaltes von Rapssamen oder Rapsschrot zu schaffen, das schnell, unkompliziert und präzise arbeitet.

Erfindungsgemäß wird diese Aufgabe durch die im Kennzeichen des Anspruchs 1 angegebenen Merkmale gelöst.

Die Unteransprüche geben vorteilhafte Ausgestaltungen des Verfahrens an.

Das vorgeschlagene Verfahren macht sich die Tatsache zu nutze, daß der Gesamtschwefelgehalt der Rapssaat und ihr Gehalt an Glucosinolaten sehr eng miteinander korrelieren, so daß der Glucosinolatgehalt indirekt über eine Gesamtschwefelanalyse bestimmt werden kann, wobei diese Gesamtschwefelanalyse dadurch durchgeführt wird, daß die Rapsprobe mit Röntgenstrahlen aus einer Röntgenröhre bestrahlt und der in der Probe befindliche Schwefel so zur Emission einer charakteristischen Eigenstrahlung (der Schwefel-$K_\alpha$-Fluorescenzstrahlung) angeregt wird. Diese Fluorescentzstrahlung wird mit Hilfe eines geeigneten Kristalls herausgefiltert und von einem Detektor gemessen (dieses Verfahren der Schwefelbestimmung ist an sich bekannt). Bei Verwendung eines in dem interessierenden Bereich ausreichend hoch auflösenden Röntgenspektrometers läßt sich der Schwefelgehalt so sehr genau bestimmen.

Aus dem Schwefelgehalt wiederum läßt sich der Gesamtglucosinolatgehalt berechnen. So ergibt sich beispielsweise ein dem durch HPLC ermittelten Gesamtglucosinolatgehalt einer Probe entsprechend der Gesamtglucosinolatgehalt aus dem berechneten Schwefelgehalt anhand einer Gleichung

$$y = 14,99 \, x - 43,9$$

ermitteln, wobei y den Gesamtglucosinolatgehalt in μmol/g lufttrockenem Samen und x den Anteil des Schwefels in der Probe in Promille angeben (Schwefel ist nicht nur in den Glucosinolaten, sondern auch in schwefelhaltigen Aminosäuren des Rapssamens enthalten). Für andere Referenzverfahren sind entsprechende andere lineare Gleichungen anzuwenden. Dasselbe gilt dann, wenn Rapsschrot untersucht werden soll.

Das vorgeschlagene Verfahren zur Bestimmung des Gesamtglucosinolatgehaltes von Rapssamen oder Rapsschrot ermöglicht den Einsatz einer relativ hohen Einwaagemenge (etwa 5 g) und sichert so im Hinblick auf Inhomogenitäten in Partien von Rapssamen repräsentative und reproduzierbare Ergebnisse. Es sind wenige und einfache Arbeitsschritte erforderlich, die bei hoher Präzision eine sichere und unkomplizierte Durchführung der Analysen ermöglichen. Der Zeitbedarf einer Analyse ist mit weniger als 2 - 4 Minuten sehr gering. Das Verfahren kann verlustfrei den Gesamtgehalt an Glucosinolaten bestimmen, es arbeitet zerstörungsfrei, so daß die Proben beliebig oft nachanalysiert werden können.

## Patentansprüche

1. Verfahren zur Bestimmung des Gesamtglucosinolatgehaltes von Rapssamen oder Rapsschrot, gekennzeichnet durch
- Vermahlen des zu untersuchenden Materials mittels eines Messerhomogenisators bis zu einer definierten Korngröße,
- Tablettieren des vermahlenen Materials bei definiertem Druck,
- Einbringen der tablettierten Probe in ein hochauflösendes Röntgenspektrometer,
- Bestrahlen der Probe mit Röntgenstrahlen,
- Messen der Schwefel-$K_\alpha$-Fluoreszenzstrahlung,
- Berechnen des Schwefelgehalts der Probe aus der Intensität der Schwefel-$K_\alpha$-Strahlen, und
- Ermitteln des Gesamtglucosinolatgehaltes aus dem berechneten Schwefelgehalt.

2. Verfahren nach Anspruch 1, dadurch gekennzeichnet, daß die definierte Korngröße über die Zeitdauer des Mahlvorgangs mittels eines bestimmten Messerhomogenisators bestimmt wird.

3. Verfahren nach Anspruch 1, dadurch gekennzeichnet, daß der definierte Druck bei dem Tablettieren durch Verwendung einer hydraulischen Handpresse erreicht wird.